# EUROPÄISCHE PATENTSCHRIFT

(11) **EP 0 266 615 B1**
(45) Veröffentlichungstag und Bekanntmachung des Hinweises auf die Patenterteilung: **28.04.1993**
(21) Anmeldenummer: 87115254.2
(22) Anmeldetag: 19.10.1987
(51) Int. Cl.: G01N 33/36

(54) **Vorrichtung zur automatischen Bestimmung von Kenngrössen von textilem Prüfgut**
Device for the automatic determination of data concerning a textile product
Appareil pour la détermination automatique de caractéristiques d'un produit textile

(30) Priorität: 07.11.1986 CH 4459/86
(43) Veröffentlichungstag der Anmeldung: 11.05.1988
(73) Patentinhaber: ZELLWEGER USTER AG, CH-8610 Uster (CH)
(72) Erfinder: Heusser, Eduard, CH-8610 Uster (CH)

(56) Entgegenhaltungen:
- WO-A-79/00324
- DE-A- 1 473 607
- FR-A- 2 244 694
- FR-A- 2 536 729
- US-A- 3 098 596
- US-A- 3 788 138
- RESEARCH DISCLOSURE, Nr. 156, April 1977, Seite 18; E.I. DU PONT DE NEMOURS & CO.: "Interlace measuring instrument";

## Beschreibung

Die Erfindung betrifft eine Vorrichtung zur automatischen Bestimmung von Kenngrössen von textilem Prüfgut, wie Garnen, Vorgarnen und Bändern, mit einer eine Führungseinrichtung, ein Messorgan, eine Vorschubeinrichtung und ein Abzugsorgan für das Prüfgut enthaltenden Messeinheit, deren Vorschubeinrichtung durch ein Paar von Transportwalzen gebildet ist.

In den Labors von Textilbetrieben, vornehmlich von Spinnereien, werden im Rahmen der betrieblichen Qualitätskontrolle Stichprobenprüfungen zur Bestimmung gewisser textiler Parameter, wie beispielsweise der Masseschwankungen und anderer davon abgeleiteter Kenngrössen vorgenommen. Dazu dienen sogenannte Gleichmässigkeitsprüfer, wie sie beispielsweise von der Anmelderin der vorliegenden Patentanmeldung unter der Bezeichnung USTER TESTER (USTER eingetragenes Warenzeichen der Zellweger Uster AG) weltweit vertrieben werden. Bei diesem bekannten Gleichmässigkeitsprüfer stellt jeder Messspalt den Luftspalt eines Kondensators dar, und die Bestimmung der genannten Kenngrössen erfolgt kapazitiv.

Zur Bestimmung der Masseschwankungen des Prüfguts wird dieses durch die Transportwalzen durch den Messkamm gezogen und nach der Prüfung durch das Abzugsorgan abgesaugt. Der Vorschub erfolgt dabei mit einer Geschwindigkeit von bis zu 400 m/min bei Prüfgut aus Stapelfasern und von bis zu 800 m/min bei Filamentgarnen. Es liegt auf der Hand, dass dabei dem einwandfreien Funktionieren der Transportwalzen eine wesentliche Bedeutung zukommt.

Bei den bekannten Gleichmässigkeitsprüfern ist eine der beiden Transportwalzen motorisch angetrieben, und der Antrieb der anderen erfolgt durch Reibungsmitnahme über die angetriebene Walze oder - bei dickem Prüfgut - über das letztere. Dadurch rotiert die nicht angetriebene Walze immer langsamer als die angetriebene. Die Differenz der Umfangsgeschwindigkeiten der beiden Walzen hängt von der Feinheit und dem Material des Prüfguts ab. Als Folge davon entstehen für den Bediener nicht erkennbare Geschwindigkeitsvariationen, die zu unzuverlässigen oder falschen Messresultaten führen.

Durch die Erfindung soll nun der bekannte Gleichmässigkeitsprüfer so verbessert werden, dass unter allen Umständen ein einwandfreier Vorschub gewährleistet ist.

Diese Aufgabe wird durch das imkennzeichnenden Teil von Anspruch 1 angegebene Merkmal gelöst.

Die erfindungsgemässe Lösung bietet den weiteren Vorteil einer möglichst schonenden Behandlung des Prüfguts, was insbesondere dann wesentlich ist, wenn am Prüfgut nach der Vorschubeinrichtung noch Messungen vorgenommen werden.

Im folgenden wird die Erfindung anhand eines Ausführungsbeispiels und der Zeichnungen näher dargestellt; es zeigen:
- Fig. 1: eine Perspektivdarstellung eines Gleichmässigkeitsprüfers zur Bestimmung der Masseschwankungen von Stapelfasergarnen,
- Fig. 2: eine Perspektivdarstellung der wesentlichen Teile der Vorschubeinrichtung der Messeinheit des Gleichmässigkeitsprüfers,
- Fig. 3: eine Frontansicht eines Teils der Messeinheit, und
- Fig. 4: eine Ansicht in Richtung des Pfeils IV von Fig. 3, bei weggelassener unterer Gehäuseplatte.

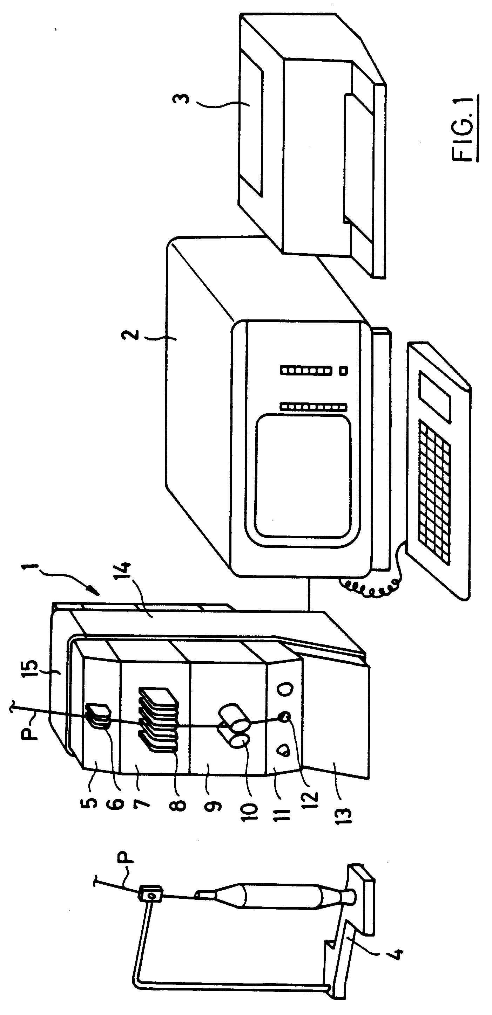

Der in Fig. 1 dargestellte Gleichmässigkeitsprüfer zur Bestimmung der Masseschwankungen von textilem Prüfgut, wie Garnen, Vorgarnen oder Bändern aus Stapelfasern, besteht darstellungsgemäss aus einer Messeinheit 1, aus einer Auswerteeinheit 2, aus einer Ausgabeeinheit 3 und aus einem Gestell 4 für die Aufmachungseinheiten mit dem Prüfgut P, das sind beispielsweise Garn- oder Vorgarnspulen. Gleichmässigkeitsprüfer dieser Art sind bekannt und werden beispielsweise von der Anmelderin der vorliegenden Patentanmeldung unter der Bezeichnung USTER TESTER (USTER eingetragenes Warenzeichen der Zellweger Uster AG) weltweit vertrieben.

Die Messeinheit 1 für das Prüfgut P besteht darstellungsgemäss aus mehreren Modulen, welche in Laufrichtung des Prüfguts P, das ist in der Figur von oben nach unten, wie folgt angeordnet sind: Zuerst ein Modul 5 mit einer Fadenführungseinrichtung 6, beispielsweise einer Fadenbremse, anschliessend ein Modul 7 mit einem Messorgan 8, anschliessend ein Modul 9 mit einer Vorschubeinrichtung 10 und anschliessend ein Modul 11 mit einer Absaugdüse 12. Das unterste Modul 11 ist auf einen Sockel 13 aufgesetzt und alle genannten Module 5, 7, 9 und 11 sowie der Sockel 13 sind in einem Rahmen 14 mit einem bügelartigen Oberteil 15 angeordnet und von diesem Rahmen gehalten.

Das Messorgan 8, durch welches das Prüfgut P durch die durch ein Walzenpaar gebildete Vorschubeinrichtung 10 gezogen wird, ist ein sogenanntes kapazitives Messorgan. Dieses ist in den US Patenten 3 754 172, 3 788 138 und 3 805 607 beschrieben, auf deren Offenbarung hiermit Bezug genommen wird. Die Absaugdüse 12 ist von dem bereits genannten USTER TESTER bekannt und wird hier nicht näher beschrieben.

Die Auswerteeinheit 2 enthält unter anderem einen Analog-/Digitalwandler und einen Rechner und ist darstellungsgemäss mit einem Bildschirm kombiniert. Die vom Messorgan 8 laufend erzeugten elektrischen Signale werden vom Rechner der Auswerteeinheit 2 verarbeitet und in geeigneter Form in einem in der Auswerteeinheit 2 integrierten Speicher gespeichert und können vor ihrem Ausdruck auf dem Ausgabeeinheit 3 bildenden Drucker auf dem Bildschirm dargestellt werden. Dies hat der Vorteil, dass man alle anfallenden Daten vorerst auf dem Bildschirm zur Anzeige bringen und nur ausgewählte Daten für den Ausdruck auf dem Drucker 3 bestimmen kann.

Es sei noch darauf hingewiesen, dass die Signalverarbeitung in der Auswerteeinheit 2 aus drei Hauptkomponenten besteht, nämlich aus dem Spektrographen, für das sogenannte Spektrogramm (Wellenlängenspektrum der Masseschwankungen), aus dem Imperfection Indicator, welcher Grenzwertüberschreitungen der Masse zählt, und aus dem eigentlichen Auswerteteil für die Bestimmung des sogenannten Variationskoeffizienten und der Längenvariationskurve. Alle diese Kenngrössen sind von dem schon genannten USTER TESTER her bekannt.

Wenn mit dem dargestellten Gleichmässigkeitsprüfer die Masseschwankungen von Filamentgarnen bestimmt werden sollen, dann muss eine Messeinheit 1 mit einem anderen Aufbau verwendet werden, welche gegenüber der in der Figur dargestellten Messeinheit 1 folgende Abweichungen aufweist:
- Es wird ein anderes Messorgan 8 benötigt, welches aber ebenfalls ein kapazitives Messorgan ist.
- Die Vorschubeinrichtung 10 muss in Laufrichtung des Prüfguts P vor dem Messorgan 8 angeordnet sein.
- Es wird eine spezielle Absaugdüse 12 benötigt, welche dem Filamentgarn den für die Prüfung erforderlichen Drall verleiht. Bezüglich dieser Absaugdüse wird hiermit auf das US-Patent Nr. 3 951 321 verwiesen.

Durch Einfügen weiterer Module in die Messeinheit 1 kann diese für die Bestimmung weiterer Parameter des Prüfguts P ausgebaut werden. So kann beispielsweise mit einem zusätzlichen Modul mit einem Messorgan für die Haarigkeit des Prüfguts P zusätzlich zu den Masseschwankungen und in einem einzigen Durchlauf des Prüfguts P durch die Messeinheit 1, dessen Haarigkeit bestimmt werden. Bezüglich dieser Ausbaumöglichkeit der Messeinheit 1 wird auf das deutsche Gebrauchsmuster 8708187 der Anmelderin der vorliegenden Patentanmeldung verwiesen.

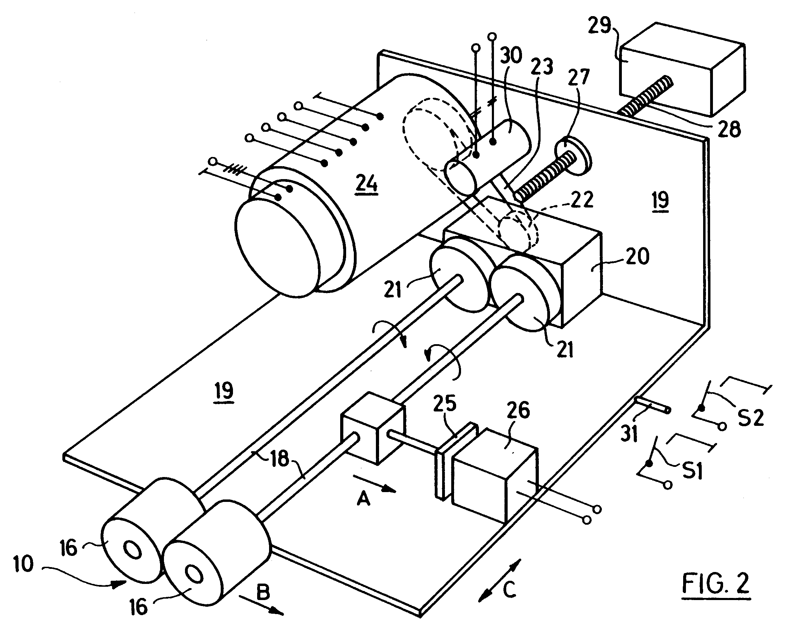

Fig. 2 zeigt eine perspektivische Prinzipdarstellung der wesentlichen Teile der Vorschubeinrichtung 10 der Messeinheit 1 des Gleichmässigkeitsprüfers von Fig. 1. Diese ist darstellungsgemäss durch ein Paar von Transportwalzen 16 aus einem hartgummiähnlichen Material gebildet, welche an der Stirnseite von zwei motorisch antreibbaren Wellen 18 montiert sind. Die Wellen 18 sind in einem auf einer L-förmigen Trägerplatte 19 montierten Lagerblock 20 gelagert und tragen je ein Zahnrad 21. Die beiden Zahnräder 21 stehen einerseits in gegenseitigem Eingriff und sind anderseits mit einer Riemenscheibe 22 verbunden, welche über einen Zahnriemen 23 mit einem Antriebsmotor 24 verbunden ist. Somit sind also beide Transportwalzen 16 formschlüssig mit dem Antriebsmotor 24 verbunden.

Die beiden Transportwalzen 16 berühren im Ruhestand einander; im Betrieb ist ihr gegenseitiger Abstand von der Dicke des Prüfguts abhängig. Das Einlegen des Prüfguts in die Messeinheit 1 (Fig. 1) erfolgt durch einen Einlegearm (nicht dargestellt), welcher das Prüfgut P ergreift und in die Fadenführungseinrichtung 6, in das Messorgan 8 und zwischen die Transportwalzen 16 einlegt und es ausserdem der Absaugdüse 12 (Fig. 1) vorlegt. Zur Erleichterung des automatischen Einlegens des Prüfguts zwischen die Transportwalzen 16 sind diese voneinander wegschwenkbar ausgebildet.

Zu diesem Zweck ist an einer der beiden Wellen 18, darstellungsgemäss an der rechten, ein stempelartiges Verstellorgan 25 befestigt, welchem ein Elektromagnet 26 zugeordnet ist und welches bei Erregung des Elektromagneten 26 von diesem angezogen wird. Dadurch wird die Welle 18 mit dem Verstellorgan 25 in Richtung des Pfeiles A bewegt, und die rechte Transportze 16 wird entsprechend in Richtung des Pfeiles B von der linken Transportwalze weggeschwenkt, wodurch zwischen den beiden Transportwalzen ein Spalt zum Einlegen des Prüfguts gebildet wird. Damit dabei die Zahnräder 21 nicht ausser Eingriff gelangen, ist zumindest die Welle 18 mit dem Verstellorgan 25, vorzugsweise jedoch beide Wellen 18 über einen Teil ihrer Länge als biegsame Welle ausgeführt. Selbstverständlich könnte anstelle der rechten auch die linke Welle 18, oder es könnten auch beide Wellen 18 verschwenkbar ausgebildet sein.

An der Rückwand der L-förmigen Trägerplatte 19 ist ein Gewindeschloss 27 drehbar gelagert, welches in Eingriff mit einer Gewindespindel 28 steht. Die letztere ist an ihrem einen Ende an einem in der Messeinheit 1 (Fig. 1) fest verankerten Bauteil 29 gehalten. Das Gewindeschloss 27 ist mit einem Antriebsmotor 30 verbunden und wird bei dessen Einschalten in Rotation versetzt. Je nach Drehrichtung bewegt sich dann die Trägerplatte 19 mit allen auf ihr befestigten und von ihr getragenen Bauteilen in Richtung des Doppelpfeiles C relativ zum Bauteil 29. Insbesondere vollführen die Transportwalzen 16 eine Hubbewegung in Richtung der Längsachsen der Wellen 18.

Die Trägerplatte 19 ist in einem Gehäuse angeordnet, welches das Modul 9 (Fig. 1) enthält, und die beiden Transportwalzen 16 ragen aus der Frontplatte dieses Gehäuses. Da das Prüfgut P zwischen der Fadenführungseinrichtung 6 und der Absaugdüse 12 (Fig. 1) gespannt ist, liegt es an einem definierten Punkt zwischen den beiden einander am nächsten liegenden Erzeugenden am Mantel der beiden Transportwalzen 16. Wenn nun die beiden Transportwalzen 16 die genannte Hubbewegung in Richtung des Pfeiles C ausführen, dann gleitet das Prüfgut entlang dieser Erzeugenden und vollführt relativ zu den Transportwalzen eine Changierbewegung. Der Fadenlauf verändert sich dadurch während des Messvorgangs nicht.

Im Betrieb der Messeinheit 1 (Fig. 1) sind die beiden Antriebsmotoren 24 und 30 ständig angetrieben, und die Transportwalzen 16 sind einerseits rotierend und anderseits in Hubrichtung (Pfeil C) angetrieben. Zur Begrenzung der Hubbewegung der Trägerplatte 19 und damit der Transportwalzen 16 ist an der Trägerplatte 19 ein seitlich abstehendes Betätigungsorgan 31 vorgesehen, in dessen Bewegungsbahn zwei Endschalter S1 und S2 angeordnet sind. Wenn nun das Gewindeschloss 27 so angetrieben wird, dass sich die Trägerplatte 19 und die Transportwalzen 16 aus der gezeichneten Lage nach vorne bewegen, dann kontaktiert das Betätigungsorgan 31 nach einem bestimmten Verstellweg den Schalter S1, durch dessen Betätigung die Drehrichtung des Antriebsmotors umgekehrt wird und Trägerplatte 19 und Transportwalzen 16 in der Gegenrichtung, also nach hinten, verstellt werden. Dies erfolgt bis zum Betätigen des Endschalters S2 durch das Betätigungsorgan 31, wodurch wiederum die Drehrichtung des Antriebsmotors 30 umgekehrt wird, und so weiter.

Dadurch erfolgt die Hubbewegung der Transportwalzen 16 und die Changierbewegung des Prüfguts ständig und vollautomatisch, wobei die Anordnung so ausgelegt ist, dass ein voller Hub, also eine Hin- und Herbewegung der Transportwalzen 16 mit einer Hublänge von etwa 25 mm pro halbem Hub in etwa vier Minuten erfolgt.

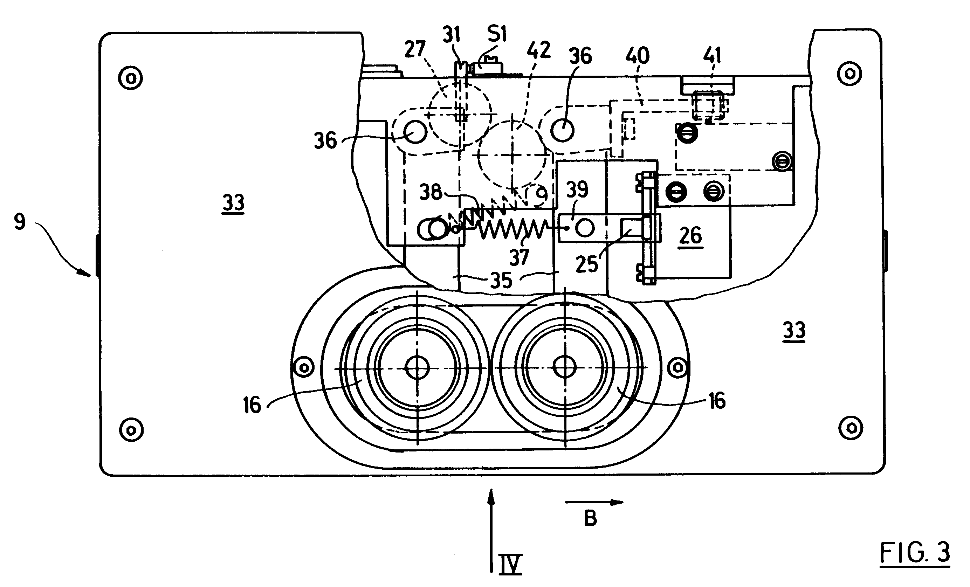

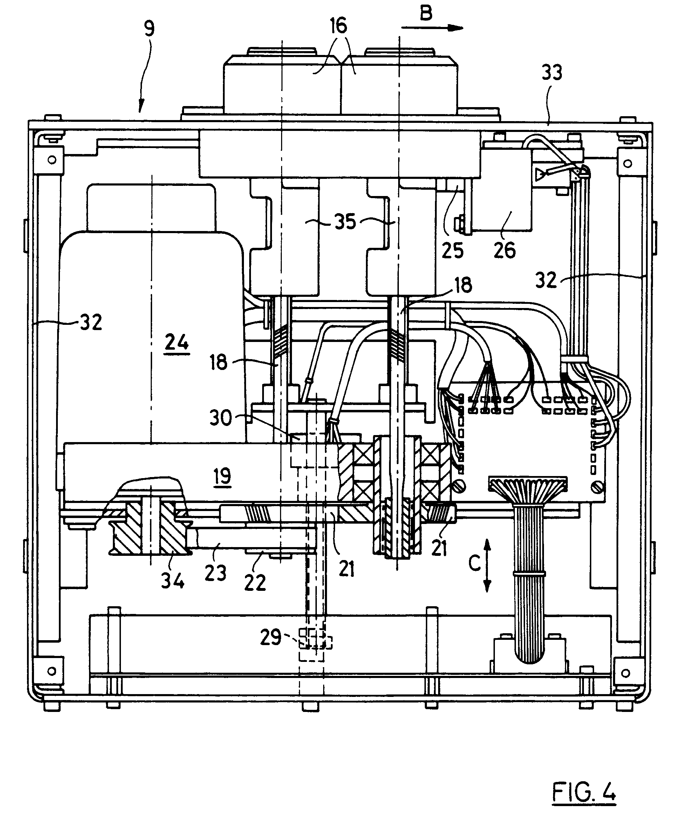

Aus den Fig. 3 und 4 ist die tatsächliche Anordnung der in Fig. 2 schematisch dargestellten Teile im Modul 9 (Fig. 1) ersichtlich. Das letztere besteht darstellungsgemäss aus einem annähernd prismatischen Gehäuse 32, welches in den Rahmen 14 (Fig. 1) eingeschoben ist und aus dessen Frontplatte 33 die beiden Transportwalzen 16 ragen.

Die Trägerplatte 19 ist als zur Frontplatte 33 parallel angeordnete, U-förmige Profilplatte ausgebildet, welche an den beiden Seitenwänden des Gehäuses 32 verschiebbar gelagert ist. Die biegsamen Wellen 18 sind in der Trägerplatte 19 gelagert und tragen an ihrem aus der Rückseite der Trägerplatte 19 ragenden Ende die beiden miteinander in Eingriff stehenden Zahnräder 21. Eine der beiden Wellen, darstellungsgemäss die rechte, trägt die Zahnriemenscheibe 22, welche über den Zahnriemen 23 von der Riemenscheibe 34 des ebenfalls auf der Trägerscheibe 19 befestigten Antriebsmotors 24 angetrieben ist.

Die beiden biegsamen Wellen 18 sind im Bereich der Frontplatte 33 je in einem Lagerarm 35 gelagert, der im Gehäuse 32 vertikal angeordnet ist. Jeder Lagerarm 35 ist an seinem oberen Ende auf einer parallel zu den Wellen 18 angeordneten Lagerwelle 36 schwenkbar gelagert und trägt an seinem unteren Ende die jeweilige Welle 18. Beide Lagerarme 35 sind je durch eine Zugfeder 37, 38 gegeneinander gespannt. Der linke Lagerarm 35 kann gegen die Kraft seiner Zugfeder 38 von Hand nach links geschwenkt werden, was bei besonders dickem Prüfgut erforderlich ist. Am rechten Lagerarm 35 ist eine Lasche 39 befestigt, welche das Verstellorgan 25 (Fig. 2) trägt. Diesem ist der Elektromagnet 26 zugeordnet, bei dessen Erregung das Verstellorgan 25 angezogen und die rechte Transportwalze 16 von der linken weggeschwenkt wird. Dies erfolgt automatisch beim Einlegen des Prüfguts P in die Messeinheit 1 (Fig. 1).

Am oberen Ende des automatisch verschwenkbaren rechten Lagerarm 35 ist ein Finger 40 befestigt, welcher bei aneinander anliegenden Transportwalzen 16` also im Prüfbetrieb, auf einen Mikroschalter 41 drückt. Sobald der den Finger 40 tragende Lagerarm 35 weggeschwenkt wird, gibt der Finger 40 den Mikroschalter 41 frei. Der Mikroschalter 41 ist ein Sicherheitsschalter, der beim Wegschwenken der rechten Transportwalze 16 im Betrieb die Antriebsmotoren 24 und 30 abschaltet. Dieses Wegschwenken im Betrieb tritt dann auf, wenn es bei nicht beaufsichtigter Messeinheit 1 und schwach eingestellter Absaugdüse 12 zu einem mehrfachen Ueberwickeln des Prüfguts auf den Transportwalzen kommt.

An der Trägerplatte 19 ist ausserdem etwa im Niveau der Lagerwellen 36 der Antriebsmotor 30 befestigt, welcher über ein Zahnrad 42 das Gewindeschloss 27 antreibt und dadurch die Hubbewegung der Trägerplatte 19 mitsamt den an dieser befestigten Bauteilen bewirkt. Die beiden im Gehäuse 32 ortfest angeordneten Endschalter S1 und S2 und deren Betätigungsorgan 31 sind aus Fig. 3 ersichtlich.

Wenn auch bei dem dargestellten Ausführungsbeispiel die Hubbewegung der Transportwalzen 16 motorisch und das Oeffnen des Spalts zwischen diesen mittels eines Elektromagneten erfolgt, so sind diese Bewegungen nicht auf die beschriebenen Antriebsarten beschränkt und es könnten selbstverständlich auch hydraulische oder pneumatische Antriebe verwendet werden. Besonders pneumatische Antriebe sind in der Textilindustrie verbreitet, so dass deren Anwendung für den Fachmann naheliegend ist.

## Patentansprüche

1. Vorrichtung zur automatischen Bestimmung von Kenngrössen von textilem Prüfgut, wie Garnen, Vorgarnen und Bändern, mit einer eine Führungseinrichtung, ein Messorgan, eine Vorschubeinrichtung und ein Abzugsorgan für das Prüfgut enthaltenden Messeinheit, deren Vorschubeinrichtung durch ein Paar von Transportwalzen gebildet ist, dadurch gekennzeichnet, dass beide Transportwalzen (16) formschlüssig mit einem Antrieb (24) verbunden sind.

2. Vorrichtung nach Anspruch 1, dadurch gekennzeichnet, dass die Transportwalzen (16) je auf einer Welle (18) montiert und die genannten Wellen formschlüssig miteinander verbunden sind und, dass eine der Wellen mit dem Antrieb (24) verbunden ist.

3. Vorrichtung nach Anspruch 2, dadurch gekennzeichnet, dass auf jeder der beiden Wellen (18) ein Zahnrad (21) montiert ist, und dass diese Zahnräder in gegenseitigem Eingriff stehen.

4. Vorrichtung nach Anspruch 3, dadurch gekennzeichnet, dass die Transportwalzen (16) federnd gegeneinander gedrückt sind.

5. Vorrichtung nach einem der Ansprüche 1 bis 4, dadurch gekennzeichnet, dass Mittel (25, 26) zur automatischen vorübergehenden Vergrösserung des Achsabstandes der beiden Transportwalzen (16) zum Einlegen des Prüfguts (P) zwischen diese vorgesehen sind.

6. Vorrichtung nach Anspruch 5, dadurch gekennzeichnet, dass die genannten Mittel durch ein an der einen Transportwalze (16) beziehungsweise an deren Träger (18) angreifendes Verstellorgan (25) und durch ein dem letzteren zugeordnetes Betätigungsorgan (26) gebildet sind.

7. Vorrichtung nach Anspruch 6, dadurch gekennzeichnet, dass das Betätigungsorgan (26) durch einen Elektromagneten gebildet ist.

8. Vorrichtung nach den Ansprüchen 2 und 7, dadurch gekennzeichnet, dass zumindest die die verstellbare Transportwalze (16) tragende Welle (18) über mindestens einen Teil ihrer Länge als biegsame Welle ausgebildet ist.

9. Vorrichtung nach Anspruch 6, dadurch gekennzeichnet, dass zum Einlegen von bandförmigem Prüfgut (P) die nicht automatisch verstellbare Transportwalze (16) von Hand von der verstellbaren wegbewegbar ausgebildet ist.

10. Vorrichtung nach Anspruch 5, dadurch gekennzeichnet, dass Mittel (40, 41) zum Abstellen des Antriebs (24) der Transportwalzen (16) bei Ueberschreiten eines bestimmten Wertes von deren gegenseitigem Achsabstand beim Durchlauf des Prüfguts (P) vorgesehen sind.

11. Vorrichtung nach den Ansprüchen 6 und 10, dadurch gekennzeichnet, dass die genannten Mittel durch ein mit der automatisch verstellbaren Transportwalze (16) gekoppeltes Betätigungselement (40) und durch einen diesem zugeordneten Schalter (41) gebildet sind.

## Claims

1. Apparatus for the automatic determination of characteristic magnitudes of textile material to be tested, such as yarns, rovings and slivers, having a measuring unit comprising a guide device, a measuring instrument, a feed device and a draw-off device for the test material, the feed device being formed by a pair of transport rollers, characterized in that both transport rollers (16) are positively connected to a drive (24).

2. Apparatus according to claim 1, characterized in that the transport rollers (16) are each mounted on a shaft (18) and the said shafts are positively connected with one another, and in that one of the shafts is connected to the drive (24).

3. Apparatus according to claim 2, characterized in that a gear wheel (21) is mounted on each of the two shafts (18), and in that these gear wheels are in engagement with one another.

4. Apparatus according to claim 3, characterized in that the transport rollers (16) are resiliently pressed against one another.

5. Apparatus according to one of claims 1 to 4, characterized in that means (25, 26) are provided for automatically temporarily increasing the distance between the axes of the two transport rollers (16) for inserting the test material (P) between them.

6. Apparatus according to claim 5, characterized in that the said means are formed by a displacement device (25) acting on one transport roller (16) or on its carrier (18) respectively, and by an actuating member (26) associated with the displacement device.

7. Apparatus according to claim 6, characterized in that the actuating member (26) is formed by an electromagnet.

8. Apparatus according to claims 2 and 7, characterized in that at least the shaft (18) carrying the displaceable transport roller (16) is a flexible shaft at least over part of its length.

9. Apparatus according to claim 6, characterized in that the transport roller (16) which is not automatically adjustable can be moved away from the adjustable transport roller by hand for the insertion of test material (P) in the form of bands.

10. Apparatus according to claim 5, characterized in that means (40, 41) are provided for switching off the drive (24) to the transport rollers (16) when the mutual axial distance exceeds a certain value at the passage of the test material (P).

11. Apparatus according to claims 6 and 10, characterized in that the said means are formed by an actuating element (40) which is coupled to the automatically adjustable transport roller (16) and by a switch (41) associated with the actuating element.

## Revendications

1. Dispositif pour la détermination automatique de caractéristiques d'un produit textile, tel que des filés, des mèches de préparation et des bandes, avec une unité de mesure comprenant un dispositif de guidage, un organe de mesure, un dispositif d'avancement et un organe de déroulement du produit textile, dont le dispositif d'avancement est constitué par une paire de cylindres transporteurs, caractérisé en ce que les deux cylindres transporteurs (16) sont reliés par concordance des formes à un entraînement (24).

2. Dispositif selon la revendication 1, caractérisé en ce que les cylindres transporteurs (16) sont montés chacun sur un arbre (18), et que lesdits arbres sont reliés l'un à l'autre par concordance des formes, et en ce que l'un des arbres est relié à l'entraînement (24).

3. Dispositif selon la revendication 2, caractérisé en ce que sur chacun des deux arbres (18) est montée une roue dentée (21), et que ces roues dentées sont en prise l'une avec l'autre.

4. Dispositif selon la revendication 3, caractérisé en ce que les cylindres transporteurs (16) sont pressés de manière élastique l'un contre l'autre.

5. Dispositif selon l'une des revendications 1 à 4, caractérisé en ce que des moyens (25, 26) sont prévus pour l'agrandissement automatique temporaire de l'écart des axes des deux cylindres transporteurs (16) pour la mise en place du produit textile (P) entre ceux-ci.

6. Dispositif selon la revendication 5, caractérisé en ce que les moyens précités sont constitués par un organe de réglage (25) venant en prise avec un cylindre transporteur (16) ou le support (18) de celui-ci et par un organe d'actionnement (26) associé à celui-ci.

7. Dispositif selon la revendication 6, caractérisé en ce que l'organe d'actionnement (26) est constitué par un électro-aimant.

8. Dispositif selon les revendications 2 et 7, caractérisé en ce qu'au moins l'arbre (18) supportant le cylindre transporteur réglable (16) est réalisé sur au moins une partie de sa longueur comme arbre flexible.

9. Dispositif selon la revendication 6, caractérisé en ce que le cylindre transporteur (16) ne pouvant pas être réglé automatiquement peut être éloigné à la main du cylindre transporteur réglable pour la mise en place du produit textile (P) en forme de bande.

10. Dispositif selon la revendication 5, caractérisé en ce que des moyens (40, 41) sont prévus pour arrêter l'entraînement (24) des cylindres transporteurs (16) lors du dépassement d'une valeur déterminée de l'écart d'axe mutuel de ceux-ci lors du passage du produit textile (P).

11. Dispositif selon les revendications 6 et 10, caractérisé en ce que les moyens précités sont constitués par un élément d'actionnement (40) couplé au cylindre transporteur (16) réglable automatiquement et par un commutateur (41) associé à celui-ci.
